# EUROPEAN PATENT APPLICATION

(11) **EP 1 225 183 A2**
(43) Date of publication of application: **24.07.2002**
(21) Application number: 02250128.2
(22) Date of filing: 09.01.2002
(51) Int. Cl.: C07K 14/705, C12N 15/63, G01N 33/53, A61K 38/00, A61K 39/39, A61K 48/00, A01K 67/027

(54) **Human G-protein coupled receptor**

(30) Priority: 23.01.2001 GB 0101739
(71) Applicant: Pfizer Limited, Sandwich, Kent CT13 9NJ (GB); PFIZER INC., New York, N.Y. 10017 (US)
(72) Inventor: Harland, Lee, Sandwich, Kent CT13 9NJ (GB)
(74) Representative: Hayles, James Richard

(57) **Abstract**

Polynucleotide and polypeptide sequences are described. The polypeptide sequences comprise one or more of (a) a polypeptide having a deduced amino acid sequence translated from the polynucleotide sequence of SEQ ID NO: 1, or a variant, fragment, homologue, analogue or derivative thereof; (b) a polypeptide of SEQ ID NO: 2, or a variant, fragment, homologue, analogue or derivative thereof.

SEQUENCE LISTING

<110> Pfizer Limited (EP (GB) only), Pfizer Inc. (EP except GB / US /
JP)

<120> Novel Polypeptide

<130> PCS10970ARFB

<150> GB0101739.1
<151> 2001-01-23

<160> 3

<170> FastSEQ for Windows Version 4.0

<210> 1
<211> 918
<212> DNA
<213> Homo sapiens

<400> 1
atgcctggcc acaatacctc caggaattcc tcttgcgatc ctatagtgac accccactta 60
atcagcctct acttcatagt gcttattggc gggctggtgg gtgtcatttc cattcttttc 120
ctcctggtga aaatgaacac ccggtcagtg accaccatgg cggtcattaa cttggtggtg 180
gtccacagcg tttttctgct gacagtgcca tttcgcttga cctacctcat caagaagact 240
tggatgtttg ggctgccctt ctgcaaattt gtgagtgcca tgctgcacat ccacatgtac 300
ctcacgttcc tattctatgt ggtgatcctg gtcaccagat acctcatctt cttcaagtgc 360
aaagacaaag tggaattcta cagaaaactg catgctgtgg ctgccagtgc tggcatgtgg 420
acgctggtga ttgtcattgt ggtacccctg gttgtctccc ggtatggaat ccatgaggaa 480
tacaatgagg agcactgttt taaatttcac aaagagcttg cttacacata tgtgaaaatc 540
atcaactata tgatagtcat ttttgtcata gccgttgctg tgattctgtt ggtcttccag 600
gtcttcatca ttatgttgat ggtgcagaag ctacgccact ctttactatc ccaccaggag 660
ttctgggctc agctgaaaaa cctatttttt ataggggtca tccttgtttg tttccttccc 720
taccagttct ttaggatcta ttacttgaat gttgtgacgc attccaatgc ctgtaacagc 780
aaggttgcat tttataacga aatcttcttg agtgtaacag caattagctg ctatgatttg 840
cttctctttg tctttggggg aagccattgg tttaagcaaa agataattgg cttatggaat 900
tgtgttttgt gccgttag 918

<210> 2
<211> 305
<212> PRT
<213> Homo sapiens

<400> 2
Met Pro Gly His Asn Thr Ser Arg Asn Ser Ser Cys Asp Pro Ile Val
1               5                   10                  15
Thr Pro His Leu Ile Ser Leu Tyr Phe Ile Val Leu Ile Gly Gly Leu
            20                  25                  30
Val Gly Val Ile Ser Ile Leu Phe Leu Leu Val Lys Met Asn Thr Arg
        35                  40                  45
Ser Val Thr Thr Met Ala Val Ile Asn Leu Val Val Val His Ser Val
    50                  55                  60
Phe Leu Leu Thr Val Pro Phe Arg Leu Thr Tyr Leu Ile Lys Lys Thr
65                  70                  75                  80
Trp Met Phe Gly Leu Pro Phe Cys Lys Phe Val Ser Ala Met Leu His
                85                  90                  95
Ile His Met Tyr Leu Thr Phe Leu Phe Tyr Val Val Ile Leu Val Thr
            100                 105                 110

Arg Tyr Leu Ile Phe Phe Lys Cys Lys Asp Lys Val Glu Phe Tyr Arg
        115                 120                 125
Lys Leu His Ala Val Ala Ala Ser Ala Gly Met Trp Thr Leu Val Ile
    130                 135                 140
Val Ile Val Val Pro Leu Val Val Ser Arg Tyr Gly Ile His Glu Glu
145                 150                 155                 160
Tyr Asn Glu Glu His Cys Phe Lys Phe His Lys Glu Leu Ala Tyr Thr
                165                 170                 175
Tyr Val Lys Ile Ile Asn Tyr Met Ile Val Ile Phe Val Ile Ala Val
            180                 185                 190
Ala Val Ile Leu Leu Val Phe Gln Val Phe Ile Ile Met Leu Met Val
        195                 200                 205
Gln Lys Leu Arg His Ser Leu Leu Ser His Gln Glu Phe Trp Ala Gln
    210                 215                 220
Leu Lys Asn Leu Phe Phe Ile Gly Val Ile Leu Val Cys Phe Leu Pro
225                 230                 235                 240
Tyr Gln Phe Phe Arg Ile Tyr Tyr Leu Asn Val Val Thr His Ser Asn
                245                 250                 255
Ala Cys Asn Ser Lys Val Ala Phe Tyr Asn Glu Ile Phe Leu Ser Val
            260                 265                 270
Thr Ala Ile Ser Cys Tyr Asp Leu Leu Leu Phe Val Phe Gly Gly Ser
        275                 280                 285
His Trp Phe Lys Gln Lys Ile Ile Gly Leu Trp Asn Cys Val Leu Cys
    290                 295                 300
Arg
305

<210> 3
<211> 337
<212> PRT
<213> Homo sapiens

<400> 3
Met Asp Glu Thr Gly Asn Leu Thr Val Ser Ser Ala Thr Cys His Asp
1               5                   10                  15
Thr Ile Asp Asp Phe Arg Asn Gln Val Tyr Ser Thr Leu Tyr Ser Met
            20                  25                  30
Ile Ser Val Val Gly Phe Phe Gly Asn Gly Phe Val Leu Tyr Val Leu
        35                  40                  45
Ile Lys Thr Tyr His Lys Lys Ser Ala Phe Gln Val Tyr Met Ile Asn
    50                  55                  60
Leu Ala Val Ala Asp Leu Leu Cys Val Cys Thr Leu Pro Leu Arg Val
65                  70                  75                  80
Val Tyr Tyr Val His Lys Gly Ile Trp Leu Phe Gly Asp Phe Leu Cys
                85                  90                  95
Arg Leu Ser Thr Tyr Ala Leu Tyr Val Asn Leu Tyr Cys Ser Ile Phe
            100                 105                 110
Phe Met Thr Ala Met Ser Phe Phe Arg Cys Ile Ala Ile Val Phe Pro
        115                 120                 125
Val Gln Asn Ile Asn Leu Val Thr Gln Lys Lys Ala Arg Phe Val Cys
    130                 135                 140
Val Gly Ile Trp Ile Phe Val Ile Leu Thr Ser Ser Pro Phe Leu Met
145                 150                 155                 160
Ala Lys Pro Gln Lys Asp Glu Lys Asn Asn Thr Lys Cys Phe Glu Pro
                165                 170                 175
Pro Gln Asp Asn Gln Thr Lys Asn His Val Leu Val Leu His Tyr Val
            180                 185                 190
Ser Leu Phe Val Gly Phe Ile Ile Pro Phe Val Ile Ile Ile Val Cys
        195                 200                 205
Tyr Thr Met Ile Ile Leu Thr Leu Leu Lys Lys Ser Met Lys Lys Asn

210                 215                 220
Leu Ser Ser His Lys Lys Ala Ile Gly Met Ile Met Val Val Thr Ala
225                 230                 235                 240
Ala Phe Leu Val Ser Phe Met Pro Tyr His Ile Gln Arg Thr Ile His
                245                 250                 255
Leu His Phe Leu His Asn Glu Thr Lys Pro Cys Asp Ser Val Leu Arg
            260                 265                 270
Met Gln Lys Ser Val Val Ile Thr Leu Ser Leu Ala Ala Ser Asn Cys
        275                 280                 285
Cys Phe Asp Pro Leu Leu Tyr Phe Phe Ser Gly Gly Asn Phe Arg Lys
    290                 295                 300
Arg Leu Ser Thr Phe Arg Lys His Ser Leu Ser Ser Val Thr Tyr Val
305                 310                 315                 320
Pro Arg Lys Lys Ala Ser Leu Pro Glu Lys Gly Glu Glu Ile Cys Lys
                325                 330                 335
Val

## Description

### Technical field

The present invention relates to a novel polynucleotide sequence which encodes a novel polypeptide belonging to the class of proteins known as G-protein coupled receptors (GPCRs). The present invention also relates, inter alia, to processes for producing the polypeptide and its uses.

### Background of the invention

Cells and tissues respond to a wide variety of extracellular signalling molecules through the interaction of these molecules with specific cell-surface receptors. One such class of receptors are known as G-protein coupled receptors (GPCRs) and these are characterised by containing a series of 7 hydrophobic transmembrane segments. Upon binding of an extracellular ligand to its receptor, intracellular signals are initiated via interactions with heterotrimeric G proteins which in turn can lead to a number of different intracellular events depending upon which receptor has been activated. For example some GPCRs influence adenyl cyclase activity whereas others act via phospholipase C.

Members of the GPCR superfamily respond to a wide variety of ligands including small molecule amines (such as serotonin, dopamine, acetylcholine), lipid-derived mediators (such as LpA), amino acid derivatives (such as glutamate) and neurotransmitter peptides and hormones (such as neurokinin, galanin, glucagon, gastrin). Although GPCRs are activated by a broad range of ligands, it should be noted that individual GPCRs have a small and very specific repertoire of ligands. Based upon an analysis of the primary structure of a novel GPCR, it is now possible to classify them into specific sub-families, thereby narrowing the range of potential ligands.

In many cases, the endogenous ligands of GPCRs are relatively small, enabling them to be mimicked or blocked by synthetic analogues. For example drugs such as prazosin, doxazosin, cimetidine, ranitidine are all effective antagonists of their respective target GPCRs.

Thus, as the activation or inhibition of GPCRs can have therapeutic consequences, there is a continued need to provide new GPCRs and their associated agonists and antagonists.

### Summary of the invention

According to one aspect of the present invention, there is provided an isolated polynucleotide comprising:
(a) a polynucleotide encoding the polypeptide as set forth in SEQ ID NO: 2;
(b) a polynucleotide comprising the nucleotide sequence of SEQ ID NO: 1;
(c) a polynucleotide comprising a nucleotide sequence that has at least 70% identity, preferably at least 70-75% identity; to the polynucleotide of (a) or (b);
(d) a polynucleotide comprising a nucleotide sequence which is capable of hybridising to a polynucleotide according to any one of (a) to (c); or
(e) a polynucleotide fragment of a polynucleotide according to any one of (a) to (d).

According to other aspects of the present invention, there is provided an isolated polynucleotide essentially consisting of, or an isolated polynucleotide consisting of, a polynucleotide according to any one of (a) to (e) above.

Preferably, the polynucleotide comprises a nucleotide sequence that has at least 75% identity, preferably at least 75-80% identity to a polynucleotide according to (a) or (b) above. More preferably, the polynucleotide comprises a nucleotide sequence that has at least 80% identity, preferably at least 80-85% identity to a polynucleotide according to (a) or (b) above. Even more preferably, the polynucleotide comprises a nucleotide sequence that has at least 85% identity, preferably at least 85-90% identity to a polynucleotide according to (a) or (b) above. More preferably, the polynucleotide comprises a nucleotide sequence that has at least 90% identity, preferably at least 90-95% identity to a polynucleotide according to (a) or (b) above. Yet more preferably, the polynucleotide comprises a nucleotide sequence that has greater than 95% identity to a polynucleotide according to (a) or (b) above. Preferably, the polynucleotide comprises a nucleotide sequence that has at least 98% identity to a polynucleotide according to (a) or (b) above.

According to another aspect of the present invention, there is provided a polypeptide comprising:
(a) a polypeptide having the deduced amino acid sequence translated from the polynucleotide sequence in SEQ ID NO: 1, and variants, fragments, homologues, analogues and derivatives thereof; or
(b) a polypeptide of SEQ ID NO: 2, and variants, fragments, homologues, analogues and derivatives thereof.

In a preferred embodiment, the polypeptide consists of SEQ ID NO: 2.

In another preferred embodiment, the polypeptide consists of a variant, fragment, homologue, analogue or derivative of SEQ ID NO: 2.

In a preferred embodiment, a polynucleotide according to the invention encodes a G-protein coupled receptor (GPCR).

The present invention also provides a polynucleotide probe or primer comprising at least 15 contiguous nucleotides of a polynucleotide according to the invention.

The present invention also provides a recombinant DNA comprising a polynucleotide sequence according to the invention.

The present invention yet further provides a vector comprising a polynucleotide according to the invention.

According to a further aspect of the present invention, there is provided a host cell comprising a vector according to the invention, preferably a host cell transformed or transfected with a vector according to the invention. Preferably, the host cell is a mammalian, insect, bacterial, fungal or yeast cell.

According to yet a further aspect of the present invention, there is provided a process for producing a polypeptide according to the invention, or fragment thereof comprising culturing said host cell comprising the vector under conditions sufficient for the expression of said polypeptide or fragment. Preferably, said polypeptide or fragment is expressed at the surface of said cell. The process preferably further includes recovering the polypeptide or fragment from the culture. There is also provided a membrane preparation of said cells.

The present invention provides a process for producing cells capable of expressing a polypeptide or fragment thereof comprising transforming or transfecting cells with a vector according to the invention.

According to a further embodiment of the present invention, there are provided cells produced by the process of transformation or transfection with a vector according to the invention. There is also provided a membrane preparation of said cells.

There is also provided by the present invention an antibody against a polypeptide according to the invention.

The present invention yet further provides a compound which activates a polypeptide according to the invention (an agonist), or a compound which inhibits activation of a polypeptide according to the invention (an antagonist).

According to another aspect of the present invention, there is provided a method for identifying an agonist compound which can bind to and activate a polypeptide according to the invention comprising:
(a) contacting the compound to be tested with cells expressing on the surface thereof a polypeptide according to the invention or a membrane preparation of said cells, said polypeptide being associated with a second component capable of providing a detectable signal in response to the binding of an agonist compound to said polypeptide; said contacting being under conditions sufficient to permit binding of agonist compounds to the polypeptide; and
(b) identifying an agonist compound capable of polypeptide binding and activation by detecting the signal produced by said second component.

According to another aspect of the present invention, there is provided a method for identifying an antagonist compound which can bind to and inhibit activation of a polypeptide according to the invention comprising:
(a) contacting (i) a detectable first component known to bind to and activate a polypeptide according to the invention and (ii) the compound to be tested with cells expressing on the surface thereof polypeptide according to the invention or a membrane preparation of said cells, said polypeptide being associated with a second component capable of providing a detectable signal in response to the binding of the detectable first component to said polypeptide; said contacting being under conditions sufficient to permit binding of the detectable first component or agonist compounds to the polypeptide; and
(b) determining the extent to which the binding of the first component to the polypeptide is affected by the test compound, by detecting the absence or otherwise of a signal generated from the interaction of the first component with the polypeptide.

As GPCRs are involved in signal transduction, agonists or antagonists of the polypeptide of the present invention can find use in interfering in, that is modulating the signal transduction process. Consequently, the present invention provides an agonist compound which can activate a GPCR polypeptide according to the invention, or an antagonist compound which can inhibit activation of a GPCR polypeptide according to the invention for use as a pharmaceutical. Agonists or antagonists of the GPCR polypeptide, can find use in therapeutic areas in which conditions and diseases are affected by signal transduction. For example, agonists or antagonists of the polypeptide, can find use in therapeutic areas such as: inflammation, allergy and respiratory, neurology, psychotherapy, urogenital disease, reproductive and sexual dysfunction/disorders, cancer, tissue repair, dermatology, skin pigmentation disorders, photoageing, frailty, osteoporosis, metabolic disease, cardiovascular disease, gastrointestinal disease, anti-infection, sensory organ disorders, sleep disorders and hairloss.

Accordingly, there is also provided the use of an agonist of a GPCR polypeptide according to the invention in the manufacture of a medicament for the treatment of a patient having a condition or disease which can be ameliorated by activation of the GPCR polypeptide.

There is also provided the use of an antagonist of a GPCR polypeptide according to the invention, in the manufacture of a medicament for the treatment of a patient having a disease or condition which can be ameliorated by inhibition of the GPCR polypeptide.

According to yet a further aspect of the invention, there is provided a method for the treatment of a patient having a disease or condition which can be ameliorated by activation of a GPCR polypeptide according to the invention, comprising administering to the patient a therapeutically effective amount of an agonist compound according to the invention. Preferably, said agonist compound is a polypeptide and a therapeutically effective amount of said polypeptide is provided by administering to the patient DNA encoding said polypeptide and expressing said polypeptide in vivo.

According to yet a further aspect of the invention, there is also provided a method for the treatment of a patient having a disease or condition that can be ameliorated by inhibition of a GPCR polypeptide according to the invention, comprising administering to the patient a therapeutically effective amount of an antagonist compound according to the invention. Preferably, said antagonist compound is a polypeptide and a therapeutically effective amount of said polypeptide is provided by administering to the patient DNA encoding said polypeptide and expressing said polypeptide in vivo.

There is also provided by the present invention a method for the treatment of a patient having need, depending on the condition or disease to be treated, either to activate or to inhibit a GPCR polypeptide according to the invention, comprising administering to the patient a therapeutically effective amount of an antibody according to the invention.

Yet further provided by the present invention is use of an antibody according to the invention in the manufacture of a medicament for the treatment of a patient having need, depending on the condition or disease to be treated, either to activate or to inhibit a GPCR polypeptide according to the invention.

According to a further aspect of the present invention, there is provided a method of treatment of a patient having need to upregulate a GPCR polypeptide according to the invention, comprising administering to the patient a therapeutically effective amount of a polypeptide according to the present invention. Preferably, said therapeutically effective amount of the polypeptide is provided by administering to the patient DNA encoding said polypeptide and expressing said polypeptide in vivo.

There is also provided by the present invention, use of a polypeptide according to the invention in the manufacture of a medicament for the treatment of a patient having need to upregulate a GPCR polypeptide according to the invention.

According to yet a further aspect of the present invention, there are provided cells or an animal genetically engineered to overexpress, underexpress or to exhibit targeted deletion of a GPCR polypeptide of the present invention.

### Detailed description of the invention

The present invention will now be described, by way of example only, with reference to the accompanying figures and sequence listing, wherein:
**Figure 1** shows the nucleotide sequence coding for PFI-021. The ATG translation initiation codon is indicated by the first three letters. The stop codon is indicated by the last three letters.
**Figure 2** shows the corresponding amino acid sequence coding for PFI-021.
**Figure 3** shows a ClustalW Alignment of PFI-021 with Cysteinyl leukotriene receptor (CYSLT1).

**SEQ ID NO: 1** is the nucleotide sequence coding for PFI-021. The ATG translation initiation codon is indicated by the first three letters. The stop codon is indicated by the last three letters.

**SEQ ID NO: 2** is the amino acid sequence for PFI-021.

**SEQ ID NO: 3** is a ClustalW Alignment of PFI-021 with cysteinyl leukotriene receptor (CYSLT1).

The polynucleotide which encodes the GPCR of the present invention was identified electronically and analysed using various bioinformatic tools. The GPCR encoded by the sequences described herein has been termed PFI-021.

The term "nucleotide sequence" as used herein refers to an oligonucleotide sequence or polynucleotide sequence, and variants, homologues, fragments and derivatives thereof (such as portions thereof). The nucleotide sequence may be DNA or RNA of genomic or synthetic or recombinant origin which may be double-stranded or single-stranded whether representing the sense or antisense strand. Preferably, the term "nucleotide sequence" means DNA. More preferably, the term "nucleotide sequence" means DNA prepared by use of recombinant DNA techniques (i.e. recombinant DNA).

In a preferred embodiment, the present invention does not cover the native nucleotide coding sequence according to the present invention in its natural environment when it is under the control of its native promoter which is also in its natural environment. For ease of reference, we shall call this preferred embodiment the "non-native nucleotide sequence".

As used herein "amino acid sequence" refers to peptide or protein sequences or portions thereof. The term "polypeptide", which is interchangeable with the term protein, refers to amino acid sequences, which are at least 5 amino acids in length, preferably at least 10 amino acids in length, more preferably at least 15 amino acids in length, yet more preferably at least 20 amino acids in length, preferably at least 50 amino acids in length.

In a preferred embodiment, the present invention does not cover the native PFI-021 polypeptide according to the present invention when it is in its natural environment and when it has been expressed by its native nucleotide coding sequence which is also in its natural environment and when that nucleotide sequence is under the control of its native promoter which is also in its natural environment. For ease of reference, we shall call this preferred embodiment the "non-native amino acid sequence".

As used herein "biologically active" refers to a PFI-021 polypeptide having structural, regulatory or biochemical functions of the naturally occurring PFI-021. A polypeptide according to the invention having "PFI-021 activity" has at least one of the structural, regulatory or biochemical functions of the native PFI-021 polypeptide. As used herein, "immunological activity" is defined as the capability of the natural, recombinant or synthetic PFI-021 peptide or any oligopeptide thereof, to induce a specific immune response in appropriate animals or cells and to bind with specific antibodies.

The term "derivative" as used herein includes chemical modification of a PFI-021 polypeptide .

As used herein, the terms "isolated" and "purified" refer to molecules, either nucleic or amino acid sequences, that are removed from their natural environment and isolated or separated from at least one other component with which they are naturally associated.

The terms "variant", "homologue" or "fragment" in relation to the amino acid sequence for the preferred polypeptide of the present invention include any substitution of, variation of, modification of, replacement of, deletion of or addition of one (or more) amino acid from or to the sequence providing the resultant polypeptide has PFI-021 activity. In particular, the term "homologue" covers homology with respect to structure and/or function.

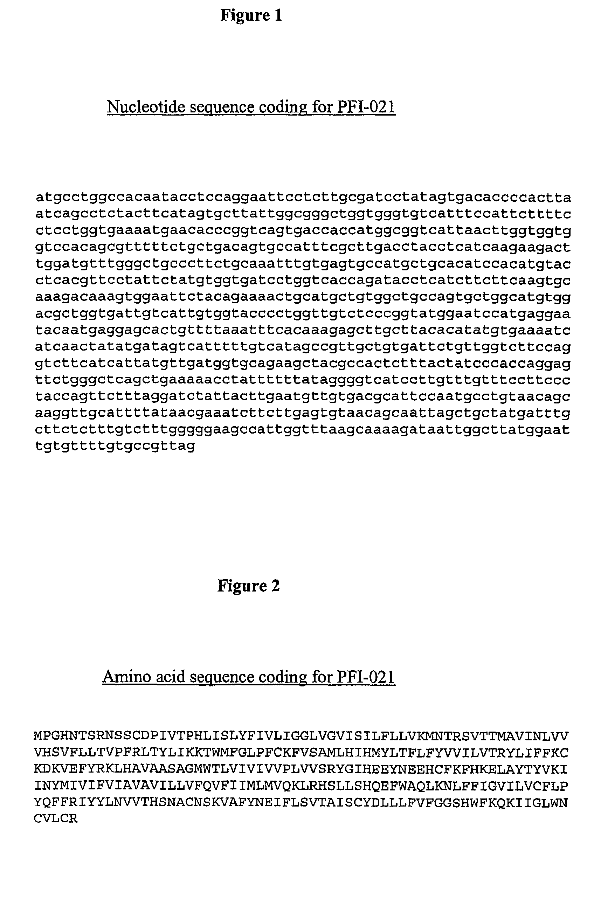

The terms "variant", "homologue" or "fragment" in relation to the nucleotide sequence coding for the preferred polypeptide of the present invention include any substitution of, variation of, modification of, replacement of, deletion of or addition of one (or more) nucleic acid from or to the sequence providing the resultant nucleotide sequence codes for or is capable of coding for a polypeptide having PFI-021 activity. In particular, the term "homologue" covers homology with respect to structure and/or function providing the resultant nucleotide sequence codes for or is capable of coding for an enzyme having PFI-021 activity. With respect to sequence homology (i.e. identity), preferably there is at least 70%, preferably at least 70-75%, more preferably at least 75%, preferably at least 75-80%, more preferably at least 80%, preferably at least 80-85%, more preferably at least 85%, preferably at least 85-90%, more preferably at least 90%, preferably at least 90-95%, yet more preferably greater than 95% identity, preferably at least 98% identity to the polynucleotide sequence shown in Figure 1 and SEQ ID NO: 1.

In particular, the term "homology" as used herein may be equated with the term "identity". Relative sequence homology (i.e. sequence identity) can be determined by commercially available computer programs that can calculate percentage (%) homology between two or more sequences. A typical example of such a computer program is CLUSTAL.

As used herein, the terms "variant", "homologue", "fragment" and "derivative" include allelic variations of the sequences.

In relation to nucleic acids, the term "variant" also encompasses sequences that are complementary to sequences that are capable of hybridising to the nucleotide sequences presented herein. Preferably, the term "variant" encompasses sequences that are complementary to sequences that are capable of hybridising, when tested under stringent conditions (e.g. 65°C and 0.1xSSC {1xSSC = 0.15 M NaCl, 0.015 Na₃ citrate pH 7.0}), to the nucleotide sequences presented herein.

The present invention also covers nucleotide sequences that can hybridise to the nucleotide sequences of the present invention (including complementary sequences of those presented herein). In a preferred aspect, the present invention covers nucleotide sequences that can hybridise to the nucleotide sequence of the present invention under stringent conditions when tested using standard laboratory techniques well known in the art (e.g. 65°C and 0.1xSSC).

The term "vector" includes expression vectors and transformation vectors.

The term "expression vector" means a construct capable of in vivo or in vitro expression.

The term "transformation vector" means a construct capable of being transferred from one species to another.

### The identification of PFI-021

PFI-021 was identified in unfinished genomic sequence information (GenBank contig accession AC083865) which is being released by the Genome Sequencing Centers by searching the sequences with known members of the G-protein coupled receptor (GPCR) family using the BLAST algorithm. In order to confirm that PFI-021 was a member of the GPCR family, a number of bioinformatics approaches were performed.

### a) BLAST Search against Swissprot

PFI-021 was searched against Swissprot using the BLAST algorithm (Basic Local Alignment Search Tool (Altschul SF (1993) J.Mol. Evol. 36:290-300; Altschul, SF et al (1990) J. Mol. Biol. 215:403-410)) to identify the closest protein match. In this case the top hit was to: Cysteinyl leukotriene receptor (CYSLT1).

These results indicate that PFI-021 is a member of the GPCR family.

### (b) ClustalW Alignment of PFI-021 with Cysteinyl leukotriene receptor (CYSLT1)

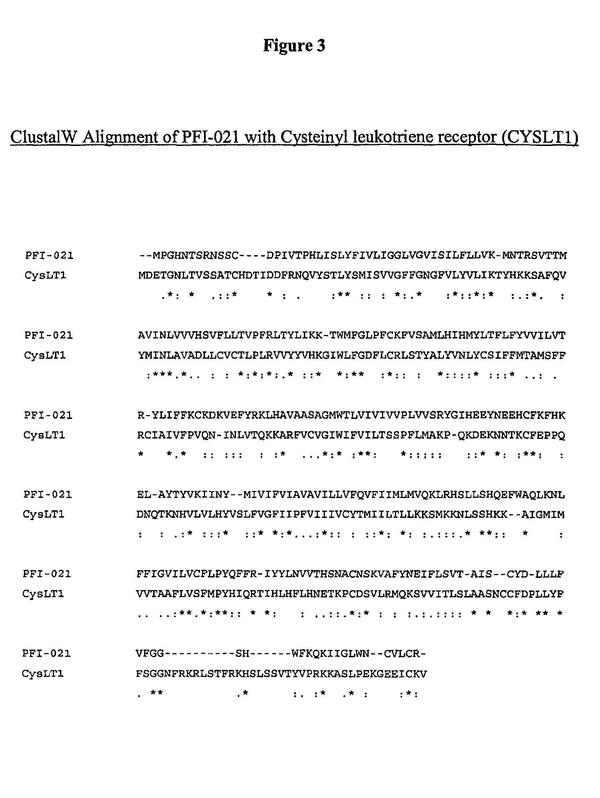

These results are shown in Figure 3.

### (c) BLAST search against a non-redundant human GPCR database

PFI-021 was searched against a non-redundant human GPCR database comprising mainly sequences from Genbank and Geneseq Patents databases in order to identify the class of agonist for this receptor. The top hits are shown below:

| **Source** | **Name** | **E-value** |
|---|---|---|
| GENBANK | Cysteinyl leukotriene receptor (CYSLT1) | 4e-20 |
| GENBANK | G protein-coupled receptor GPR34 [L:381] | 1e-18 |
| GENESEQP | G-protein coupled receptor GRIR-2 (gene... | 7e-18 |
| SWISSPROT | G protein-coupled receptor GPR18 (frag... | 7e-18 |
| Genbank | Cysteinyl Leukotriene receptor CysLT2 (P... | 8e-17 |
| SWISSPROT | P2Y purinoceptor 5 (P2Y5) [L:344] | 1e-16 |
| SWISSPROT | G protein-coupled receptor KIAA0001. U... | 7e-16 |
| SWISSPROT | G protein-coupled receptor GPR7. [L:328] | 1e-15 |
| (e value = statistical likelihood of the hit occurring by chance) | | |

These results demonstrate that PFI-021 is most closely similar to cysteinyl leukotriene receptors and they suggest that PFI-021 encodes a novel GPCR whose ligand is likely to be a cysteinyl leukotriene.

### (d) Tissue distribution of PFI-021

Expressed sequence tags of the PFI-021 sequence are found mainly in cDNA libraries prepared from peripheral blood mononuclear cells; two ESTs have also been identified in libraries prepared from breast mRNA.

It will be appreciated that the foregoing is provided by way of example only and modification of detail may be made without departing from the scope of the invention.

## Claims

1. An isolated polynucleotide essentially consisting of:
(a) a polynucleotide encoding a polypeptide as set forth in SEQ ID NO: 2;
(b) the nucleotide sequence of SEQ ID NO: 1;
(c) a nucleotide sequence that has at least 70% identity to the polynucleotide of (a) or (b);
(d) a polynucleotide consisting of a nucleotide sequence which is capable of hybridising to a polynucleotide according to any one of (a) to (c); or
(e) a polynucleotide fragment of a polynucleotide according to any one of (a) to (d).

2. A polynucleotide according to claim 1, comprising a nucleotide sequence that has at least 75% identity to a polynucleotide according to (a) or (b).

3. A polynucleotide according to claim 1, comprising a nucleotide sequence that has at least 80% identity to a polynucleotide according to (a) or (b).

4. A polynucleotide according to claim 1, comprising a nucleotide sequence that has at least 85% identity to a polynucleotide according to (a) or (b).

5. A polynucleotide according to claim 1, comprising a nucleotide sequence that has at least 90% identity to the polynucleotide according to (a) or (b).

6. A polynucleotide according to claim 1, comprising a nucleotide sequence that has greater than 95% identity to a polynucleotide according to (a) or (b).

7. A polynucleotide according to claim 1, comprising a nucleotide sequence that has greater than 98% identity to a polynucleotide according to (a) or (b).

8. A polynucleotide according to any one of the preceding claims which encodes a G-protein coupled receptor (GPCR).

9. A polynucleotide probe or primer comprising at least 15 contiguous nucleotides of a polynucleotide according to any one of the preceding claims.

10. A recombinant DNA comprising a polynucleotide sequence according to claim 1.

11. A vector comprising a polynucleotide according to any one of claims 1 to 8.

12. A host cell comprising a polynucleotide according to any one of claims 1 to 8.

13. A host cell transformed or transfected with a vector according to claim 11.

14. A host cell according to claim 12 or claim 13 which is a mammalian, insect, bacterial, fungal, or yeast cell.

15. A polypeptide comprising:
(a) a polypeptide having the deduced amino acid sequence translated from the polynucleotide sequence in SEQ ID NO: 1, and variants, fragments, homologues, analogues and derivatives thereof; or
(b) the polypeptide of SEQ ID NO: 2, and variants, fragments, homologues, analogues and derivatives thereof.

16. A polypeptide consisting of SEQ ID NO: 2.

17. A polypeptide consisting of a variant, fragment, homologue analogue or derivative of the polypeptide of SEQ ID NO: 2.

18. A process for producing a polypeptide according to any one of claims 15 to 17 comprising culturing the host cell according to any one of claims 12 to 14 under conditions sufficient for the expression of said polypeptide.

19. A process according to claim 18, wherein said expressed polypeptide is present at the surface of said cell.

20. A process according to claim 18 or claim 19 which further includes recovering the polypeptide from the culture.

21. A process for producing cells capable of expressing a polypeptide according to any one of claims 15 to 17 comprising transforming or transfecting cells with a vector according claim 11.

22. Cells produced by the process of claim 21.

23. A membrane preparation of the cells according to claim 22.

24. An antibody against a polypeptide according to any one of claims 15 to 17.

25. An agonist which activates a GPCR polypeptide which corresponds to a polypeptide according to any one of claims 15 to 17.

26. An antagonist which inhibits activation of a GPCR polypeptide which corresponds to a polypeptide according to any one of claims 15 to 17.

27. A method for identifying an agonist compound which can bind to and activate a GPCR polypeptide which corresponds to a polypeptide according to any one of claims 15 to 17 comprising:
(a) contacting a compound to be tested with cells expressing on the surface thereof a polypeptide according to any one of claims 15 to 17, or a membrane preparation of said cells, said polypeptide being associated with a second component capable of providing a detectable signal in response to the binding of an agonist compound to said polypeptide; said contacting being under conditions sufficient to permit binding of agonist compounds to the polypeptide; and
(b) identifying an agonist compound capable of polypeptide binding and activation by detecting the signal produced by said second component.

28. A method for identifying an antagonist compound which can bind to and inhibit activation of a GPCR polypeptide which corresponds to a polypeptide according to any one of claims 15 to 17 comprising:
(a) contacting (i) a detectable first component known to bind to and activate a polypeptide according to any one of claims 15 to 17 and (ii) a compound to be tested with cells expressing on the surface thereof a polypeptide according to any one of claims 15 to 17, or a membrane preparation of said cells, said polypeptide being associated with a second component capable of providing a detectable signal in response to the binding of said detectable first component to said polypeptide; said contacting being under conditions sufficient to permit binding of said detectable first component or agonist compounds to the polypeptide; and
(b) determining the extent to which the binding of the first component to the polypeptide is affected by the test compound, by detecting the absence or otherwise of a signal generated from the interaction of the first component with the polypeptide.

29. An agonist according to claim 25 or an antagonist according to claim 26 for use as a pharmaceutical.

30. Use of an agonist according to claim 25 in the manufacture of a medicament for the treatment of a patient having a condition or disease which can be ameliorated by activation of the GPCR.

31. Use of an antagonistaccording to claim 26 in the manufacture of a medicament for the treatment of a patient having a condition or disease which can be ameliorated by inhibition of the GPCR.

32. A method for the treatment of a patient having a condition or disease which can be ameliorated by activation of a GPCR corresponding to a polypeptide according to any one of claims 15 to 17 comprising administering to the patient a therapeutically effective amount of an agonist according to claim 25.

33. The method of claim 32, wherein said agonist is a polypeptide and a therapeutically effective amount of the compound is provided by administering to the patient DNA encoding said agonist polypeptide and expressing said agonist polypeptide in vivo.

34. A method for the treatment of a patient having a condition or disease which can be ameliorated by inhibition of a GPCR corresponding to a polypeptide according to any one of claims 15 to 17 comprising administering to the patient a therapeutically effective amount of an antagonist according to claim 26.

35. The method of claim 34, wherein said antagonist is a polypeptide and a therapeutically effective amount of the antagonist polypeptide is provided by administering to the patient DNA encoding said antagonist polypeptide and expressing said antagonist polypeptide in vivo.

36. A method for the treatment of a patient having a condition or disease which can be ameliorated by either activation of, or inhibition of, a GPCR corresponding to a polypeptide according to any one of claims 15 to 17, comprising administering to the patient a therapeutically effective amount of an antibody according to claim 24.

37. Use of an antibody according to of claim 24 in the manufacture of a medicament for the treatment of a patient having a condition or disease which can be ameliorated by either the activation or the inhibition of a GPCR corresponding to a polypeptide according to any one of claims 15 to 17.

38. A method of treatment of a patient having need to upregulate a GPCR corresponding to a polypeptide according to any one of claims 15 to 17, comprising administering to the patient a therapeutically effective amount of a polypeptide according to any one of claims 15 to 17.

39. The method of claim 38, wherein said therapeutically effective amount of polypeptide is provided by administering to the patient DNA encoding said polypeptide and expressing said polypeptide in vivo.

40. Use of a polypeptide according to any one of claims 15 to 17 in the manufacture of a medicament for the treatment of a patient having need to upregulate a GPCR corresponding to a polypeptide according to any one of claims 15 to 17.

41. Cells or animal genetically engineered to overexpress a polypeptide according to any one of claims 15 to 17.

42. Cells or animal genetically engineered to underexpress a polypeptide according to any one of claims 15 to 17.

43. Cells or animal genetically engineered to exhibit targeted deletion of a polypeptide according to any one of claims 15 to 17.
